# EUROPÄISCHE PATENTSCHRIFT

(11) **EP 3 346 962 B1**
(45) Veröffentlichungstag und Bekanntmachung des Hinweises auf die Patenterteilung: **22.05.2019**
(21) Anmeldenummer: 16763263.7
(22) Anmeldetag: 09.09.2016
(51) Int. Cl.: A61F 5/453, A61F 6/00

(54) **VORRICHTUNG ZUM ANLEGEN EINES URINALKONDOMS ZUR BESTIMMUNGSGEMAESSEN VERWENDUNG**
DEVICE FOR APPLYING A CONDOM FOR ITS PROPER USE
DISPOSITIF POUR L'APPLICATION D'UN CONDOM POUR L'UTILISATION CORRECTE

(30) Priorität: 10.09.2015 DE 102015115296; 16.10.2015 DE 102015117685
(43) Veröffentlichungstag der Anmeldung: 18.07.2018
(73) Patentinhaber: Ehrensperger, Samuel M., 40545 Düsseldorf (DE)
(72) Erfinder: Ehrensperger, Samuel M., 40545 Düsseldorf (DE)
(74) Vertreter: Rausch Wanischeck-Bergmann Brinkmann
(86) Internationale Anmeldenummer: PCT/EP2016/071324
(87) Internationale Veröffentlichungsnummer: WO 2017/042344

(56) Entgegenhaltungen:
- CH-A5- 672 591
- DE-A1- 4 322 858
- GB-A- 2 278 279
- US-A- 2 567 926
- US-A- 5 471 998

## Beschreibung

Die vorliegende Erfindung betrifft eine Vorrichtung zum Anlegen eines Urinalkondoms zur bestimmungsgemäßen Verwendung.

Eine mit zunehmenden Überalterung der Gesellschaften ebenfalls zunehmende Behinderung ist die sogenannte Inkontinenz, auch als Blasenschwäche bezeichnet. Unkontrolliert auslaufende Flüssigkeit wird entweder mittels saugender Hilfsmittel aufgenommen, beispielsweise Windeln und dergleichen, oder abgeleitet. Zur Ableitung werden Katheter verwendet, die entweder durch die Bauchdecke direkt in die Blase eingesetzt werden, was häufig zu Infektionen führen kann, oder durch die Harnröhre des Penis in die Blase geschoben werden und zu Verletzungen an der Harnröhre führen können. Eine Alternative sind sogenannten Urinalkondome, die eine Verbindung mit einem abführenden Schlauch, mit Auffangbeuteln und dergleichen ermöglichen.

Für die Versorgung der männlichen Harninkontinenz stellen Urinalkondome eine bevorzugte Alternative dar. Ein Nachteil besteht darin, dass das Anlegen eines Urinalkondoms schwierig sein kann. Bei eingeschränkter Fingerfertigkeit muss dies durch Fremdpersonen, Pflegepersonal oder dergleichen erfolgen. Aus unterschiedlichsten Gründen erweist sich dabei das ein Kondom aufnehmende Glied oftmals als wenig aufnahmefähig. Dies führt häufig zu Undichtigkeiten oder Faltungen, schlechten Sitz und dergleichen. Betroffene Patienten mit retrahiertem Penis oder mit Impotenz ist es bisher überhaupt nicht möglich, Urinalkondome anzusetzen und zu benutzen.

Aus dem Stand der Technik ist aus der CH 672 591 A5 eine Vorrichtung zum Ansetzen eines Urinals bekannt. Die Vorrichtung ermöglicht das vereinfachte Ansetzen eines Urinals, in dem die Penis-Manschette des Urinals über den Rand der Öffnung eines becherförmigen Gefäßes dieser Vorrichtung gestülpt wird und im Inneren dieses Gefäßes ein Unterdruck erzeugt wird. Dadurch bläht sich die am Auslassende verschlossene Manschette auf und erlaubt einen problemlosen Zugang in das Innere.

Ausgehend vom vorbeschriebenen Stand der Technik liegt der vorliegenden Erfindung die **Aufgabe** zugrunde, eine Vorrichtung zum Anlegen eines Urinalkondoms zur bestimmungsgemäßen Verwendung vorzuschlagen, welche das Anlegen eines Urinalkondoms für nahezu alle Patienten ermöglicht, die darüber hinaus einen guten Sitz eines Urinalkondoms sicherstellt und ein Anlegen generell erheblich vereinfacht.

Zur technischen **Lösung** wird eine Vorrichtung mit den Merkmalen des Patentanspruches 1 vorgeschlagen. Weitere Vorteile und Merkmale ergeben sich aus den Unteransprüchen.

Die erfindungsgemäße Vorrichtung umfasst eine Aufnahmeglocke, eine Vakuumpumpe und eine Verbindungseinheit zur strömungstechnischen Verbindung der Aufnahmeglocke mit der Vakuumpumpe.

Als Aufnahmeglocke wird ein topfförmiger Vorrichtungsteil bezeichnet, in welchen ein Urinalkondom einsetzbar ist. Urinalkondome liegen in der Regel in aufgerollter Form vor. Sie haben an der Spitze einen schlauchartigen Ansatz. Die Aufnahmeglocke wird so ausgestaltet, dass sie für die jeweilige Größe eines Urinalkondoms eine Möglichkeit zum Einlegen des Kondoms bietet, welches sich an die glockenförmige innere Oberfläche der Aufnahmevorrichtung anlegt. Der schlauchartige Ansatz ragt durch eine Öffnung an der entsprechenden Stelle der Aufnahmeglocke heraus.

Eine Verbindungseinheit hat einen rohrförmigen Kanal, so dass dann, wenn die Aufnahmeglocke mit der Verbindungseinheit verbunden ist, der Schlauchansatz eines eingelegten Kondoms in den Kanal ragt. Die Verbindungseinheit ist wiederum mit einer Vorrichtung zur Erzeugung eines Unterdruckes verbunden. Dies kann ein Pumpball oder eine Pumpschere sein, die manuell betätigt werden, oder eine Elektropumpe für den Netzbetrieb oder Batteriebetrieb. Im einfachsten Fall ist die Verbindungseinheit lediglich ein Schlauch, der an der Aufnahmeglocke derart befestigt ist, dass er den Schlauchansatz eines eingesetzten Kondoms aufnehmen kann und die Vakuumpumpe strömungstechnisch mit dem Innenraum der Aufnahmegtocke verbindet.

Um das Anlegen zu optimieren, ist die Innenkontur der Aufnahmeglocke so ausgearbeitet, dass sie ein Urinalkondom einer vorgegebenen Größe passgenau aufnehmen kann. Dadurch wird Faltenbildung vermieden und ein optimales Ansetzen unterstützt. An der bestimmungsgemäß äußeren Oberkante, das heißt die freie Oberkante die nicht mit einer Verbindungseinheit verbunden wird, weist die Aufnahmeglocke gemäß einem vorteilhaften Vorschlage der Erfindung eine umlaufende Nut auf. Diese dient dem Zweck, den Kondomring, der sich durch das Aufrollen des Urinalkondoms ergibt, aufzunehmen, wenn das Urinalkondom in die Aufnahmeglocke eingesetzt wird. Im Falle, dass keine Nut ausgebildet ist, ist erfindungsgemäß auch vorgesehen, dass der Kondomring an den Rändern der Aufnahmeglocke übergestülpt oder beispielsweise mit einem Klemmring befestigt wird.

In vorteilhafter Weise ist die Aufnahmeglocke außenseitig zylindrisch, um für eine gute Handhabung eine entsprechende Haptik bereitzustellen.

Um für unterschiedliche Urinalkondome einsetzbar zu sein, kann die Aufnahmeglocke austauschbar mit der Verbindungseinheit verbunden werden. Auf diese Weise kann die Verbindungseinheit mit Aufnahmeglocken unterschiedlicher Größe und Ausbildung verbindbar sein.

Die gesamte Vorrichtung kann mit einem integrierenden Gehäuse versehen sein, in welches beispielsweise eine batteriebetriebene Elektropumpe, ein Verbindungskanal und eine Aufnahmeglocke untergebracht sind, wobei die Aufnahmeglocke in einem lösbaren Gehäuseteil angeordnet sein kann.

Für die bestimmungsgemäße Verwendung wird in die Aufnahmeglocke ein Urinalkondom derart eingesetzt, dass der Schlauchansatz durch eine untere Öffnung der Aufnahmeglocke in den rohrförmigen Kanal der Verbindungseinheit ragt und der Kondomring an der freien Oberkante der Aufnahmeglocke zum anliegen kommt. Der dazwischenliegende Kondombereich legt sich an den glockenförmig ausgebildeten Innenbereich der Aufnahmeglocke an. Die so vorbereitete Vorrichtung wird nun bestimmungsgemäß an einen Penis herangeführt und dieser wird aufgrund des durch den Betrieb der Vakuumpumpe erzeugten Unterdrucks in den an der Aufnahmeglocke anliegenden Bereich des Urinalkondoms eingesogen. Dabei werden Größen, Weichheiten und dergleichen durch einfaches Einsaugen kompensiert, so dass das Urinalkondom optimal angesetzt ist. Durch Abschalten der Vakuumpumpe kann nun die Vorrichtung abgenommen werden, so dass das Kondom am Glied verbleibt. Es kann nun in bestimmungsgemäßer Weise einfach abgerollt werden und ist somit optimal angesetzt. Auch ist ein Abnehmen der Vorrichtung bei laufender Vakuumpumpe, die unter Umständen regelbar ist, möglich, so dass in bestimmten Fällen auch ein faltenfreies Abrollen des Urinalkondoms unterstützt wird.

Mit der Erfindung wird eine einfach herstellbare und vor allem einfach anwendbare Vorrichtung bereitgestellt, mit welcher das Anlegen eines Urinalkondoms zur bestimmungsgemäßen Verwendung erheblich vereinfacht und optimiert wird.

Weitere Vorteile und Merkmale ergeben sich aus der folgenden Beschreibung anhand der Figuren. Dabei zeigen:
- Fig. 1: eine schematische Darstellung eines typischen Urinalkondoms;
- Fig. 2: eine schematische Darstellung einer Aufnahmeglocke und einer Verbindungseinheit;
- Fig. 3: eine Darstellung gemäß Fig. 2 mit eingesetztem Urinalkondom;
- Fig. 4: eine Darstellung des Ansetzvorganges der erfindungsgemäßen Vorrichtung zum Anlegen des Kondoms zur bestimmungsgemäßen Verwendung;
- Fig. 5: eine Darstellung gemäß Fig. 4 in einer fortgeführten Position;
- Fig. 6: eine Darstellung gemäß Fig. 4 in einer abschließenden Position und
- Fig. 7: eine Darstellung des Urinalkondoms in bestimmungsgemäßer Position.

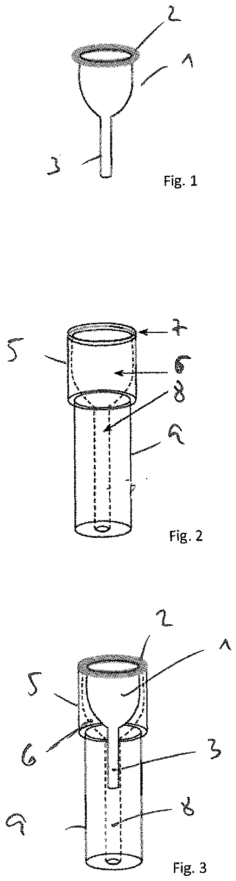

Fig. 1 zeigt ein typisches Urinalkondom 1. Dieses besteht aus einem elastischen Material und bildet eine Hülle, die ringförmig aufgerollt ist. Am vorderen Ende des Urinalkondoms befindet sich ein Schlauchansatz. Die Aufwicklung 2 dient der Vereinfachung des Anlegens des Kondoms, der Schlauchansatz 3 der Abführung.

In den Figuren 2 und 3 ist ein Ausführungsbeispiel für eine erfindungsgemäße Vorrichtung gezeigt. Eine Aufnahmeglocke 5 weist einen inneren glockenförmigen Hohlraum 6 auf. Dieser hat vorzugsweise eine Form, die der Aufnahme eines Urinalkondoms in der vorgesehenen Größe entspricht. Eine an der Oberkante umlaufende Nut 7 dient der Aufnahme des aufgewickelten Bereiches des Urinalkondoms, dem sogenannten Kondomring. Die Aufnahmeglocke 5 ist im Inneren strömungstechnisch verbunden mit einem rohrförmigen Kanal 8 der Verbindungseinheit 9. Die Aufnahmeglocke 5 und die Verbindungseinheit 9 sind außenseitig zylindrisch ausgebildet. Eine strömungsdichte Verbindung zwischen Aufnahmeglocke 5 und Verbindungseinheit 9 kann auf jede herkömmliche Weise hergestellt werden, verpressen, verschrauben, unter Verwendung von Dichtringen und dergleichen.

Wie insbesondere Fig. 3 zeigt, lässt sich in die Vorrichtung ein Urinalkondom derart einsetzen, dass der nicht aufgewickelte Bereich in der Aufnahmeglocke aufgenommen wird und der Schlauchansatz 3 in den rohrförmigen Kanal eingesetzt ist.

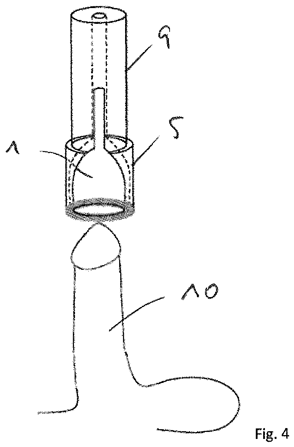

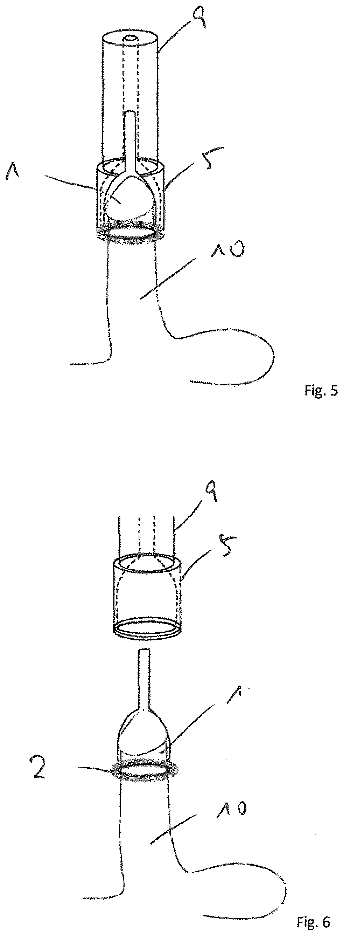

Die Applikation ist in den Figuren 4 bis 6 gezeigt. Die mit einer nicht gezeigten Vorrichtung zur Erzeugung eines Unterdrucks, Vakuumpumpe genannt, verbundene Verbindungseinheit stellt sicher, dass beim Aufsetzen der Vorrichtung auf einen Penis 10 das Urinalkondom 1 nach dem Aufsetzen auf die Spitze ein Einsaugen zulässt, während es sich selbst an der Aufnahmeglocke abstützt. Der Unterdruck kann manuell oder elektrisch erzeugt werden. Nach dem Einsaugen des Penis 10 in das Urinalkondom 1, wie dies in Fig. 5 gezeigt ist, kann die Vorrichtung abgenommen werden. Eine entsprechend höhere Haftreibung bewirkt, dass das Kondom aus der Aufnahmeglocke abgenommen wird. Dies kann durch Abschalten des Unterdrucks unterstützt werden.

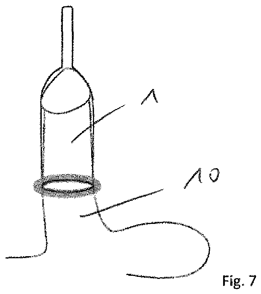

Wie in Fig. 7 gezeigt, ist nunmehr nur noch ein übliches Abrollen des aufgerollten Kondombereiches erforderlich.

Das beschriebene Ausführungsbeispiel dient nur der Erläuterung und ist nicht beschränkend.

### Bezugszeichen

- 1: Urinalkondom
- 2: Aufwicklung
- 3: Schlauchansatz
- 5: Aufnahmeglocke
- 6: Hohlraum
- 7: Nut
- 8: rohrförmiger Kanal
- 9: Verbindungseinheit
- 10: Penis

## Patentansprüche

1. Vorrichtung zum Anlegen eines Urinalkondoms (1) zur bestimmungsgemäßen Verwendung, mit einer Aufnahmeglocke (5), einer Vakuumpumpe und einer Verbindungseinheit (9) zur strömungstechnischen Verbindung der Aufnahmeglocke (5) mit der Vakuumpumpe, wobei die Verbindungseinheit (9) einen rohrförmigen Kanal (8) aufweist, mit dem die Aufnahmeglocke (5) strömungstechnisch verbunden ist, dadurch gekennnzeichnet, dass der rohrförmige Kanal (8) aufnahmeglockenseitig als Aufnahme für einen Schlauchansatz des Urinalkondoms ausgebildet ist.

2. Vorrichtung nach Anspruch 1, **dadurch gekennzeichnet, dass** die Aufnahmeglocke (5) eine zur passgenauen Aufnahme eines Urinalkondoms (1) ausgearbeitete Innenkontur aufweist.

3. Vorrichtung nach einem der vorhergehenden Ansprüche, **dadurch gekennzeichnet, dass** die Aufnahmeglocke (5) an einer Oberkante eine umlaufende Nut (7) aufweist.

4. Vorrichtung nach einem der vorhergehenden Ansprüche, **dadurch gekennzeichnet, dass** die Aufnahmeglocke (5) außenseitig zylindrisch ist.

5. Vorrichtung nach einem der vorhergehenden Ansprüche, **dadurch gekennzeichnet, dass** die Aufnahmeglocke (5) lösbar mit der Verbindungseinheit (9) verbunden ist.

6. Vorrichtung nach einem der vorhergehenden Ansprüche, **dadurch gekennzeichnet, dass** die Verbindungseinheit (9) im Wesentlichen zylindrisch ausgearbeitet ist.

7. Vorrichtung nach einem der vorhergehenden Ansprüche, **dadurch gekennzeichnet, dass** die Aufnahmeglocke (5) und die Verbindungseinheit (9) aus Kunststoff bestehen.

8. Vorrichtung nach einem der vorhergehenden Ansprüche, **dadurch gekennzeichnet, dass** die Vakuumpumpe manuell oder elektrisch betreibbar ist.

9. Vorrichtung nach einem der vorhergehenden Ansprüche, **dadurch gekennzeichnet, dass** die Vakuumpumpe und die Verbindungseinheit (9) in ein gemeinsames Gehäuse integriert sind.

## Claims

1. A device for applying a urinal condom (1) for the proper use thereof, comprising a receiving cover (5), a vacuum pump and a connection unit (9) for the fluidic connection of the receiving cover (5) and the vacuum pump, wherein the connection unit (9) comprises a tubular channel (8) with which the receiving cover (5) is connected in a fluidic manner, **characterized in that** the tubular channel (8) is configured on the side of the receiving cover as an element receiving a hose attachment of the urinal condom.

2. A device according to claim 1, **characterized in that** the receiving cover (5) comprises an inner contour which is prepared for the custom-fit reception of a urinal condom (1).

3. A device according to one of the preceding claims, **characterized in that** the receiving cover (5) comprises a circumferential groove (7) at a top edge.

4. A device according to one of the preceding claims, **characterized in that** the outside of the receiving cover (5) is cylindrical.

5. A device according to one of the preceding claims, **characterized in that** the receiving cover (5) is connected with the connection unit (9) in a detachable manner.

6. A device according to one of the preceding claims, **characterized in that** the connection unit (9) is essentially formed cylindrically.

7. A device according to one of the preceding claims, **characterized in that** the receiving cover (5) and the connection unit (9) are made of plastic.

8. A device according to one of the preceding claims, **characterized in that** the vacuum pump can be manually or electrically operated.

9. A device according to one of the preceding claims, **characterized in that** the vacuum pump and the connection unit (9) are integrated in a common housing.

## Revendications

1. Dispositif destiné à appliquer un préservatif urinaire (1) pour l'utilisation correcte, comprenant une cloche de réception (5), une pompe à vide et une unité de connexion (9) pour relier la cloche de réception (5) de manière fluidique à la pompe à vide, l'unité de connexion (9) comprenant un canal tubulaire (8) auquel est reliée la cloche de réception (5) de manière fluidique, **caractérisé en ce que** sur le côté de la cloche de réception le canal (8) tubulaire est configuré pour recevoir une fixation de tuyau du préservatif urinaire.

2. Dispositif selon la revendication 1, **caractérisé en ce que** la cloche de réception (5) comprend un contour intérieur configuré pour recevoir un préservatif urinaire (1) avec adaptation optimale.

3. Dispositif selon l'une des revendications précédentes, **caractérisé en ce que** la cloche de réception (5) comprend une rainure circonférentielle (7) sur un bord supérieur.

4. Dispositif selon l'une des revendications précédentes, **caractérisé en ce que** l'extérieur de la cloche de réception (5) est cylindrique.

5. Dispositif selon l'une des revendications précédentes, **caractérisé en ce que** la cloche de réception (5) est reliée à l'unité de connexion (9) de manière amovible.

6. Dispositif selon l'une des revendications précédentes, **caractérisé en ce que** l'unité de connexion (9) est essentiellement configurée en forme cylindrique.

7. Dispositif selon l'une des revendications précédentes, **caractérisé en ce que** la cloche de réception (5) et l'unité de connexion (9) sont fabriquées en plastique.

8. Dispositif selon l'une des revendications précédentes, **caractérisé en ce que** la pompe à vide peut fonctionner manuellement ou électriquement.

9. Dispositif selon l'une des revendications précédentes, **caractérisé en ce que** la pompe à vide et l'unité de connexion (9) sont intégrées dans un boîtier commun.
